Europäisches Patentamt

European Patent Office

Office européen des brevets

(11) Publication number: **0 506 632 A2**

# EUROPEAN PATENT APPLICATION

(21) Application number: **92830150.6**

(22) Date of filing: **25.03.92**

(51) Int. Cl.5: **C12N 15/87**

(30) Priority: **28.03.91 IT RM910205**

(43) Date of publication of application:
**30.09.92 Bulletin 92/40**

(84) Designated Contracting States:
**AT BE DE DK ES FR GB GR NL PT SE**

(71) Applicant: **Ente per le nuove tecnologie,
l'energia e l'ambiente ( ENEA)
Viale Regina Margherita 125
I-00198 Roma(IT)**

(72) Inventor: **Broglia, Marinella, c/o Enea-Ente per
le Nuove Te.
l'Energia el'Ambiente, Viale Regina
Margherita 125
I-00198 Rome(IT)**

(74) Representative: **de Simone, Domenico et al
Ing. Barzanò & Zanardo Roma S.p.A. Via
Piemonte 26
I-00187 Roma(IT)**

(54) **Process for laser cell microporation.**

(57) An automatic continuous laser cell microporation process is described, said process allowing exogenous material to be incorporated and possibly the genetic transformation of receptor cells with very high yields, said process being preferably suitable for employment in the transformation of germinal cells.

An apparatus for carrying out the process of the invention as mentioned above is also disclosed.

FIG. 1

This invention relates to a process for laser cell microporation.

More particularly, this invention relates to a process for laser microporation for introducing exogenous material into cells, both into the cytoplasmic and in the nuclear compartment.

Numerous processes are already known in the prior art for introducing exogenous material, and in particular DNA, into cells, such processes leading sometimes to the integration of exogenous DNA in the genome of the host cell. Generally, methods of chemical modifications of the cell membrane are employed, as well as "manual" methods by means of microinjection apparatus and methods exploiting electrical fields (electrofusion and electroporation). Each one of said methods shows variable efficiencies and yields. In the case of chemical methods, the transfer efficiency is very low (of the order of one cell over 100,000) and in addition the high concentration of chemical compounds employed is quite easily toxic for cells. Manual microinjection methods show on the contrary a transfer probability at least 100 times as much, but they require a very high technical skill and they result in a very low yield. As regards electrical methods for electrofusion of different cells or electroporation of the cell membrane in order to make the introduction of exogenous genetic material easier, no statistical data can be found in the technical literature which can be compared homogeneously with those mentioned above.

In particular, plant cells, which in addition to the cell membrane also have a strong wall of cellulose material, cannot be directly transformed by the techniques mentioned above. To that aim, some enzymatic digestion procedures of the wall and regeneration of the wall itself after transformation of the protoplast (the plant cell without its wall) have been devised. However, such procedures can damage the cells so treated, and can be even ineffectual in the treatment of plants belonging to the family of Gramineae, of the monocotyledons class, to which all genera of the highest agronomical importance belong (for instance, wheat, rice, maize, barley, oats, sorghum), of the highest alimentary and economic bearing. Bacterial techniques and the so-called biolysis techniques have been devised in the attempt at transforming intact plant cells complete with their walls. Agrobacterium tumefaciens is widely employed at present as a vector of exogenous genetic material inside plant cells, but with success just in the case of species of the dicotyledon class. The technique of bombarding a tissue or a cell suspension with tungsten microprojectiles after wetting the same in the solution of DNA to introduce into the cells is very recent.

The application of laser technology to such field has already been put into evidence by some authors. In the European patent application EP 137504 an apparatus is described for laser microporation of cells and for transfection with exogenous DNA. Tau et al., 1987, Proc. Natl. Acad. Sci. USA, 84: 4180-4184, describe a method for transfection of DNA into human cells in a culture by means of laser perforation. Finally, Weber et al., 1988, Plant Cell, Tissue and Organ Culture, 12: 219-222, disclose such procedure as applied to plant cells.

The advantages of laser perforation with respect to the traditional transfection procedures consist above all in a higher efficiency of the process and in increased yields, even by one order of magnitude, with respect to microinjection. This is due to the high precision of the laser beam as well as to the automation possibilities of the technique itself. Such procedures are more accurate and selective with respect to the techniques consisting in bombarding cells with microprojectiles mentioned above.

Due to the very easy way by which plant cells are able to regenerate in vitro the whole plant (somatic embryogenesis), their genetic transformation can be made inevitable. In the present state, agronomical species like those mentioned above cannot be treated by means of such procedures, but they could be transformed genetically employing as cells the germinal cells (pollen, ovules) which are then employed for in vitro or in vivo fertilizations. In case the amount of transformed cells is very low (for instance, an amount obtained by manual microinjection), fertilization is to be carried out in vitro: and as a consequence further factors intervene so as to reduce the efficiency of the process and/or vitality of the small plant, once the same has been transferred in field conditions. On the other side, the technique of pollen incubation in a germinal medium containing the transforming DNA, would allow the obtainment of sufficient amounts for in vivo pollination, but up to the present time it has given no satisfying results. Pollen laser microporation, a process carried out till the present time in static and unautomated systems, has required hypertonic osmotic conditions which favour the mechanical introduction of exogenous material following to microporation, conditions which are quite often lethal for the pollen granules. On the other side, the amount of pollen that can be treated manually by means of such technique does not allow the employment of fertilization techniques other than in vitro techniques, and in addition does not allow a statistical analysis of the power of the technique itself to be performed.

Accordingly, the need for a laser cell microporation procedure is evident, such procedure being applicable also to cells endowed with a wall like plant cells and being capable of overcoming the problems of low efficiencies and yields, so as to allow the transformation under non-lethal osmotic conditions of large amounts of cells to be carried out, and accordingly so as to allow a statistical study of the power of the technique itself to be carried out. The amounts of transformed cells which can be obtained allow in addition the cell selection to be performed by means of standard cytometric procedures and, in the case of germinal cells, they also allow in vivo fertilization procedures to be carried out.

Accordingly, it is an object of this invention an automatic and continuous cell laser microporation process, which operates under conditions of monocellular flow, wherein the detection of the passage of each cell gives a signal that causes a synchronized emission of the laser pulse for microporation of the cell itself.

According to the present invention, cells are suspended in a carrying fluid, which preferably contains the exogenous material to be introduced into the cells themselves. The motion of cells can make the introduction of said exogenous material into the cells themselves easier.

Again according to the present invention, said exogenous material comprises genetic material like plasmid vectors, DNA viral vectors or RNA viral vectors, which contain foreign genetic information, and proteins material as well.

The apparatus that realizes the process according to this invention comprises cell flow means, means for detecting the passage of each single cell, means for generating single laser pulses for microporation of said cells, and synchronization means.

Again according to this invention, said detection means comprise optical means, said optical means comprise a laser source or a source of conventional light, and means for detecting optical density changes or light scattering changes.

Again according to this invention, said means for generating single laser pulses comprise any laser source, preferably short-pulse lasers in the visible or ultra-violet spectral bands.

Said synchronization means comprise means for regulating the flowrate of cells as a function of cell sizes, with a correction as a function of intrinsic delays; the concentration of cells treated has an upper limit equal to the limit imposed by the maximum value of the repetition frequency of said means for generating laser pulses.

Said exogenous material comprises fluorescent molecular probes capable of selecting successively the cells that have been actually transformed, by means of flow cytometres.

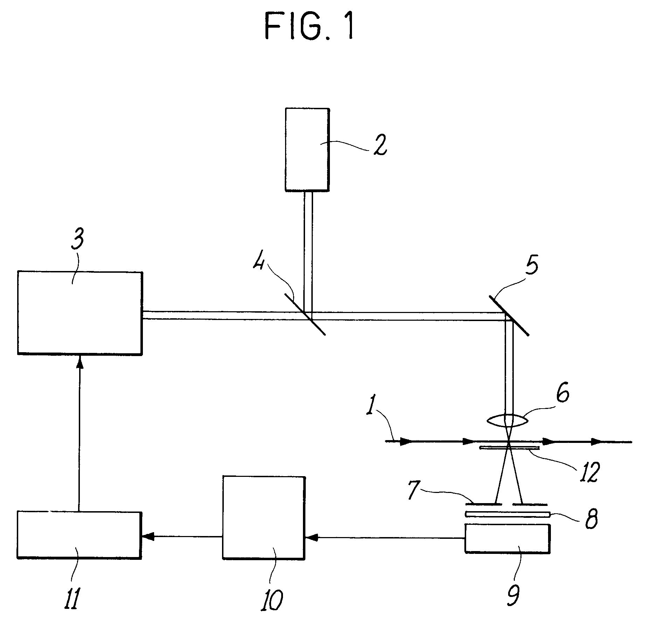

This invention will be disclosed in the following with reference to Figure 1 which shows the scheme of an apparatus that realizes the process according to the present invention, and also with reference to the following example which is supplied for explanation purposes.

With reference now to Figure 1, the flow system 1 is realized by injecting in a controlled way a suspension of maize pollen, said suspension (not shown) being stirred magnetically, into a glass microcapillary tube arranged on the microscope stage 12.

The red beam of a continuous helium-neon (He-Ne) laser 2 and a beam coming from a neodimium-yttrium aluminum garnet (Nd-YAG) short-pulse laser 3 whose frequency is triplicated, are co-linearly aligned in the microscope by means of a beam combiner 4 and of a mirror 5 and are focused by the objective length 6 onto the inside flow volume. A light detector comprising the photomultiplier 9 screened by means of spatial filters 7 and optical filters 8 is inserted below the microscope stage 12 for detecting the transit of single pollen granules at the interception point with the red beam from the laser 2.

The electric signal caused by the granule shade on the photomultiplier is amplified by an amplifier 10 and is employed by a pulse generator 11 for giving a trigger signal for emission of an ultraviolet pulse from the laser 3 for microporation of the granule itself.

In that specific case, the average diameter of the pollen granules is of about 100 $\mu$m and, employing a 40 x objective lens, cracks of some $\mu$m have been produced which, under static conditions, were shown to self-heal within 0.1-20 seconds, according to the osmotic conditions employed.

The flowrate was adjusted so as to obtain a shade signal in the detector of about 10 ms and synchronization was easily realized by delaying the trigger pulse in a suitable way. The duration of the laser impulse was of about 10 ns and the maximul repetition frequency was of 20 Hz.

This invention has been disclosed with specific reference to some preferred embodiments of the same, but it is to be understood that modifications and/or changes can be introduced in tha same by those who are skilled in the art without departing from the spirit and scope of the invention for which a priority right is claimed.

## Claims

1. A process for laser cell microporation, characterized in that it is automatic and operates continuously under conditions of monocellular flow, and wherein the detection of the transit of each cell supplies a signal that causes the synchronized emission of the laser pulse for microporation of the cell itself.

2. A process for laser cell microporation according to claim 1, said process comprising the steps of:
   - preparing a suspension of cells in a fluid
   - passing said fluid continuously through a flow means arranged on the stage of a microscope under conditions of monocellular flow
   - detecting the transit of each single cell
   - supplying, through said detection a signal that causes the synchronized emission of the laser pulse for microporation of the cell itself.

3. A process for laser cell microporation according to any one of the preceding claims, characterized in that said monocellular flow is realized by resuspending cells throughout a carrier fluid.

4. A process for laser cell microporation according to claim 3, characterized in that said carrier fluid also contain exogenous material to be introduced into the cells themselves, and also characterized in that the motion of cells makes the introduction of said exogenous material into the cells themselves easier.

5. A process for laser cell microporation according to claim 4, characterized in that said exogenous material is included in the group consisting of genetic material (DNA, RNA) and protein-kind material.

6. A process for laser cell microporation according to claim 5, characterized in that said genetic material comprises plasmid vectors or DNA viral vectors or RNA viral vectors containing foreign genetic information.

7. A process for laser cell microporation according to claim 6, characterized in that said genetic material has been previously labelled with fluorescent molecular probes, so as to select successively the cells that have been actually transformed by means of flow cytometres.

8. A process for laser cell microporation according to any one of the preceding claims, characterized in that said cells are eukaryotic cells.

9. A process for laser cell microporation according to any one of the preceding claims 1-7, characterized in that said cells are prokaryotic cells.

10. A process for laser cell microporation according to claim 8, characterized in that said eukaryotic cells are plant cells.

11. A process for laser cell microporation according to claim 10, characterized in that said plant cells belong to the class of monocotyledons.

12. A process for laser cell microporation according to claim 11, characterized in that said cells belong to the family of Gramineae.

13. A process for laser cell microporation according to any one of the preceding claims 8-12, characterized in that said cells are germinal cells or precursors thereof.

14. A process for laser cell microporation according to claim 13, characterized in that said cells are male gametophytes.

15. An apparatus for carrying out the process of laser cell microporation according to any one of the preceding claims, said apparatus being characterized in that it comprises:
    - continuous flow means for the flow a cell suspension
    - means for detecting the transit of single cells
    - means for generating automatically synchronized laser pulses.

16. An apparatus for realizing the process of laser cell microporation according to claim 15, characterized in that said means for detecting the transit of single cells include a continuous laser source or a conventional light source and a light detector, so as to signal the transit of cells and generate an electric signal.

17. An apparatus for carrying out the process of laser cell microporation according to claim 16, characterized in that said means for generating automatically sunchronized laser pulses comprise a pulse generator which is connected to said light detector by means of a signal amplifier.

18. An apparatus for carrying out the process of laser cell microporation according to claim 17, characterized in that it comprises flow cytometry means for selecting cells which actually incorporate said exogenous material.

# FIG. 1